# EUROPEAN PATENT APPLICATION

(11) **EP 4 403 090 A1**
(43) Date of publication of application: **24.07.2024**
(21) Application number: 21957457.1
(22) Date of filing: 14.09.2021
(51) Int. Cl.: A61B 1/045, G06T 7/00

(54) **IMAGE PROCESSING DEVICE, IMAGE PROCESSING METHOD, AND STORAGE MEDIUM**

(71) Applicant: NEC Corporation, 108-8001 Tokyo (JP)
(72) Inventor: SAIKOU, Masahiro, Tokyo 108-8001 (JP)
(74) Representative: Betten & Resch
(86) International application number: PCT/JP2021/033785
(87) International publication number: WO 2023/042273

(57) **Abstract**

The image processing device 1X includes an acquisition means 30X, a mask image generation means 33X, and a selection means 34X. The acquisition means 30X acquires time series images obtained by photographing an examination target by a photographing unit provided in an endoscope. The mask image generation means 33X generates a plurality of mask images, which indicate candidate regions for an attention part with different levels of granularity, for each of the time series images. Then, the selection means 34X selects an output image for output use from the time series images, based on the plurality of mask images,

## Description

### TECHNICAL FIELD

The present disclosure relates to a technical field of an image processing device, an image processing method, and storage medium for processing images acquired in endoscopic examination.

### BACKGROUND

An endoscopic system for displaying images taken in the lumen of an organ is known. For example, Patent Literature 1 discloses a learning method of a learning model configured to output information relating to a lesion part included in a captured image data when the captured image data generated by the photographing device is inputted. Further, Non-Patent Literature 1 discloses Feature Pyramid Networks which is an architecture of a neural network for inference using multi-scale feature maps. In addition, Non-Patent Literature 2 discloses a guideline in the United Kingdom relating to sampling of a biopsy part.

### CITATION LIST

### PATENT LITERATURE

Patent Literature 1: WO2020/003607

### NON-PATENT LITERATURE

Non-Patent Literature 1: T.-Y. Lin, P. Dollar, R. Girshick, K. He, B. Hariharan, and S. Belongie, Feature pyramid networks for object detection, In CVPR, 2017.
Non-Patent Literature 2: Rebecca C Fitzgerald, Massimiliano di Pietro, Krish Ragunath, et al., British Society of Gastroenterology guidelines on the diagnosis and management of Barrett's oesophagus, <https://www.bsg.org.uk/wp-content/uploads/2019/12/BSG-guidelines-on-the-diagnosis-and-management-of-Barretts-oesophagus.pdf>, October 28, 2013, P17.

### SUMMARY

### PROBLEM TO BE SOLVED

When detecting a region of an attention part such as a lesion part from an image taken in an endoscopic examination, there is an issue that detection by an examiner or a CAD (Automated Diagnosis) is difficult depending on a target attention part (e.g., a flat lesion part). Besides, among images taken during the endoscopic examination, there are images with various characteristics due to the removal of the endoscope, treatment, and the like. For example, images taken during endoscopy include images with shine, images with noise due to splash, blurred images, and the like.

In view of the above-described issue, it is therefore an example object of the present disclosure to provide an image processing device, an image processing method, and a storage medium capable of accurately selecting an image to be used for output from images captured in an endoscopic examination.

### MEANS FOR SOLVING THE PROBLEM

One mode of the image processing device is an image processing device including:
an acquisition means configured to acquire time series images obtained by photographing an examination target by a photographing unit provided in an endoscope;
a mask image generation means configured to generate a plurality of mask images, which indicate candidate regions for an attention part with different levels of granularity, for each of the time series images; and
a selection means configured to select an output image for output use from the time series images, based on the plurality of mask images.

One mode of the image processing method is an image processing method executed by a computer, the image processing method including:
acquiring time series images obtained by photographing an examination target by a photographing unit provided in an endoscope;
generating a plurality of mask images, which indicate candidate regions for an attention part with different levels of granularity, for each of the time series images; and
selecting an output image for output use from the time series images, based on the plurality of mask images.

One mode of the storage medium is a storage medium storing a program executed by a computer, the program causing the computer to:
acquire time series images obtained by photographing an examination target by a photographing unit provided in an endoscope;
generate a plurality of mask images, which indicate candidate regions for an attention part with different levels of granularity, for each of the time series images; and
select an output image for output use from the time series images, based on the plurality of mask images.

### EFFECT

An example advantage according to the present invention is to suitably select an output image regarding an attention part from time series images obtained by photographing an examination target by a photographing unit provided in an endoscope.

### BRIEF DESCRIPTION OF THE DRAWINGS

[FIG. 1] It illustrates a schematic configuration of the endoscope examination system.
[FIG. 2] It illustrates a hardware configuration of an image processing device.
[FIG. 3] FIG. 3A is a schematic diagram of a classification model built based on the classification model information.
   FIG. 3B is a schematic diagram of a mask image generation model built based on mask image generation model information.
[FIG. 4] It is a functional block diagram of an image processing device.
[FIG. 5] FIG. 5A is a diagram schematically illustrating a method of determining a lesion classifying period based on a first example.
   FIG. 5B is a diagram schematically illustrating a method of determining a lesion classifying period based on a second example.
[FIG. 6] It illustrates an outline of the processes performed by the mask image generation unit and the image selection unit.
[FIG. 7] It illustrates an outline of calculating the number of similar mask images using mask images of a past captured image.
[FIG. 8] It illustrates a first display example of the display screen image displayed by a display device in the endoscopic examination.
[FIG. 9] FIG. 9A illustrates the outline of the process of determining the biopsy part map by the selection of the mask image.
FIG. 9B illustrates the outline of the process of determining the biopsy part map by the integration of mask images.
[FIG. 10] It illustrates a second display example of the display screen image displayed by the display device in the endoscopic examination.
[FIG. 11] It is an example of a flowchart showing an outline of a process performed by the image processing device during the endoscopic examination in the first example embodiment.
[FIG. 12] It is a schematic configuration diagram of an endoscopic examination system in a modification.
[FIG. 13] It is a block diagram of an image processing device according to a second example embodiment.
[FIG. 14] It is an example of a flowchart executed by the image processing device in the second example embodiment.

### EXAMPLE EMBODIMENTS

Hereinafter, example embodiments of an image processing device, an image processing method, and a storage medium will be described with reference to the drawings.

### <First Example Embodiment>

### (1) System Configuration

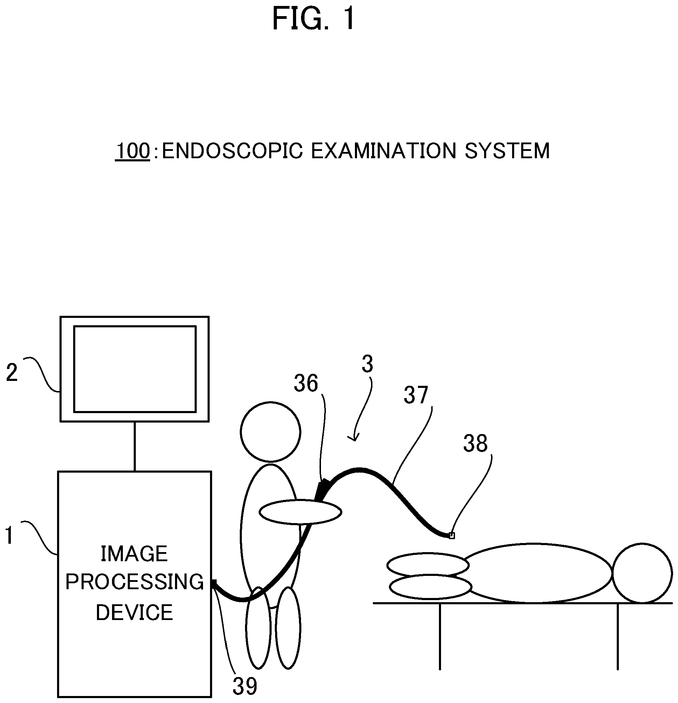

FIG. 1 shows a schematic configuration of an endoscopic examination system 100. As shown in FIG. 1, an endoscopic examination system 100 is a system configured to present a candidate for a biopsy part to an examiner who conducts examination or treatment using an endoscope, and mainly includes an image processing device 1, a display device 2, and an endoscope 3 connected to the image processing device 1. The biopsy part is a target part of a biopsy (biological tissue sampling examination). In other words, the biopsy part indicates a part suspected of a lesion. It is noted that examples of the biopsy part include not only a part suspected of a lesion but also include a peripheral region of the above-mentioned part and any other part where a biopsy is determined to be necessary. The biopsy part is an example of the "attention part".

The image processing device 1 acquire images (also referred to as "captured images Ia") captured by the endoscope 3 in time series from the endoscope 3 and displays a screen image based on the captured images Ia on the display device 2. The captured images Ia are images captured at predetermined time intervals in at least one of the insertion process of the endoscope 3 to the subject or the ejection process of the endoscope 3 from the subject. In the present example embodiment, the image processing device 1 analyzes the captured images Ia to identify a biopsy part from the captured images Ia and displays information regarding the identified biopsy part on the display device 2.

The display device 2 is a display or the like for displaying information based on the display signal supplied from the image processing device 1.

The endoscope 3 mainly includes an operation unit 36 for an examiner to perform a predetermined input, a shaft 37 which has flexibility and which is inserted into the organ to be photographed of the subject, a tip unit 38 having a built-in photographing unit such as an ultrasmall image pickup device, and a connecting unit 39 for connecting to the image processing device 1.

In the following description, as a representative example, the process in the endoscopic examination of a large bowel will be described, but the examination target may be not only the large bowel but also an esophagus or a stomach. Examples of the target endoscope in the present disclosure include a laryngendoscope, a bronchoscope, an upper digestive tube endoscope, a duodenum endoscope, a small bowel endoscope, a large bowel endoscope, a capsule endoscope, a thoracoscope, a laparoscope, a cystoscope, a cholangioscope, an arthroscope, a spinal endoscope, a blood vessel endoscope, and an epidural endoscope.

### (2) Hardware Configuration

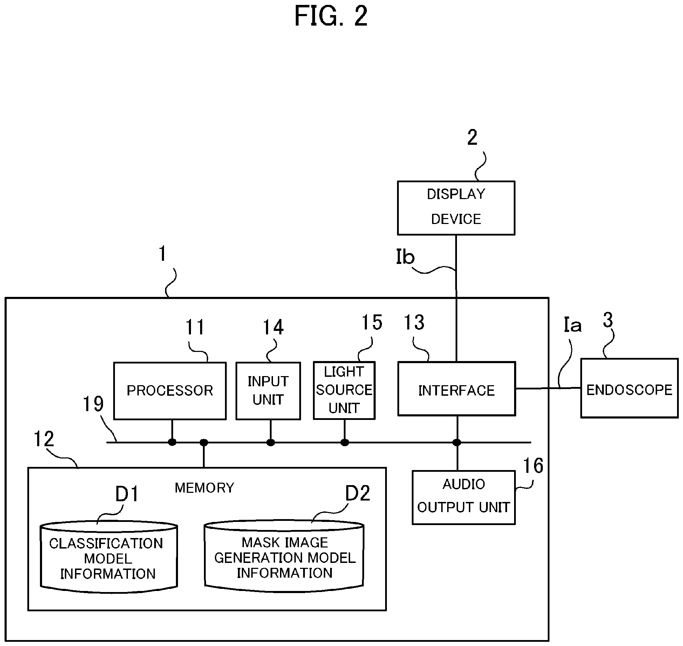

FIG. 2 shows a hardware configuration of the image processing device 1. The image processing device 1 mainly includes a processing device 11, a memory 12, an interface 13, an input unit 14, a light source unit 15, and an audio output unit 16. Each of these elements is connected to one another via a data bus 19.

The processing device 11 executes a predetermined process by executing a program or the like stored in the memory 12. The processor 11 is a processor such as a CPU (Central Processing Unit), a GPU (Graphics Processing Unit), and a TPU (Tensor Processing Unit). The processor 11 may be configured by a plurality of processors. The processor 11 is an example of a computer.

The memory 12 is configured by various memories including volatile memories used as working memories and non-volatile memories for storing the information necessary for the image processing device 1, such as a RAM (Random Access Memory) and a ROM (Read Only Memory). The memory 12 may include an external storage device, such as a hard disk, that is connected to or embedded in the image processing device 1, or may include a storage medium, such as a removable flash memory. The memory 12 stores a program for the image processing device 1 to execute the process according to the present embodiment. Further, the memory 12 stores classification model information D1 and mask image generation model information D2.

The classification model information D1 is information regarding a classification model configured to output information regarding the classification as to whether or not a captured image Ia includes a lesion region. The mask image generation model information D2 is information regarding a model (also referred to as "mask image generation model") configured to generate mask images each of which is an image indicating a biopsy part in a captured image Ia. Here, the mask image may be a binary image in which the presence or absence of the biopsy part is indicated for each grid (which refers to a unit block consisting of a pixel or a plurality of pixels) or may be an image (so-called reliability map) showing the reliability of presence of a biopsy part on a scale of three or more levels.

At least one of the classification model information D1 and/or the mask image generation model information D2 may be stored in an external device capable of data communication by wire or wirelessly with the image processing device 1 instead of the memory 12. The external device may be one or more server devices.

The interface 13 performs an interface operation between the image processing device 1 and an external device. For example, the interface 13 supplies the display information "Ib" generated by the processor 11 to the display device 2. Further, the interface 13 supplies the light generated by the light source unit 15 to the endoscope 3. The interface 13 also provides an electrical signal to the processor 11 indicative of the captured image Ia supplied from the endoscope 3. The interface 13 may be a communication interface, such as a network adapter, for wired or wireless communication with the external device, or a hardware interface compliant with a USB (Universal Serial Bus), a SATA (Serial AT Attachment), or the like.

The input unit 14 generates an input signal based on the operation by the examiner. Examples of the input unit 14 include a button, a touch panel, a remote controller, and a voice input device. The light source unit 15 generates light for supplying to the tip unit 38 of the endoscope 3. The light source unit 15 may also incorporate a pump or the like for delivering water and air to be supplied to the endoscope 3. The audio output unit 16 outputs a sound under the control of the processor 11.

### (3) Model Information

Next, the classification model information D1 and the mask image generation model information D2 will be described.

The classification model information D1 is information regarding the classification model configured to output information regarding the classification as to whether or not the captured image Ia includes a lesion region, and includes parameters required to build the classification model. The classification model is a machine learning model or a statistical model, and is a model configured to output, when a captured image Ia is inputted thereto, information (classification information) regarding whether or not a lesion region exists in the inputted captured image Ia. The classification model may output a binary value (e.g., a value of 0 or 1) depending on whether or not a lesion region is present, may output a set of a confidence level of the presence of the lesion region and a confidence level of the absence of the lesion region, or may output both of them. The "confidence level of the presence of the lesion region" is a value indicating the degree of possibility that the lesion region is present in the captured image Ta, and it increases with an increase in the degree of possibility. The "confidence level of the absence of the lesion region" is a value indicating the degree of the possibility that the lesion region does not exist in the captured image Ia, and it increases with an increase in the degree of possibility.

The condition of the lesion region to be detected by the classification model is exemplified as (a) to (f) below.
(a) Head and neck: pharyngeal cancer, malignant lymphoma, papilloma
(b) Esophagus: esophageal cancer, esophagitis, esophageal hiatal hernia, Barrett's esophagus, esophageal varices, esophageal achalasia, esophageal submucosal tumor, esophageal benign tumor
(c) Stomach: gastric cancer, gastritis, gastric ulcer, gastric polyp, gastric tumor
(d) Duodenum: duodenal cancer, duodenal ulcer, duodenitis, duodenal tumor, duodenal lymphoma
(e) Small bowel: small bowel cancer, small bowel neoplastic disease, small bowel inflammatory disease, small bowel vascular disease
(f) Large bowel: colorectal cancer, colorectal neoplastic disease, colorectal inflammatory disease; colorectal polyps, colorectal polyposis, Crohn's disease, colitis, intestinal tuberculosis, hemorrhoids

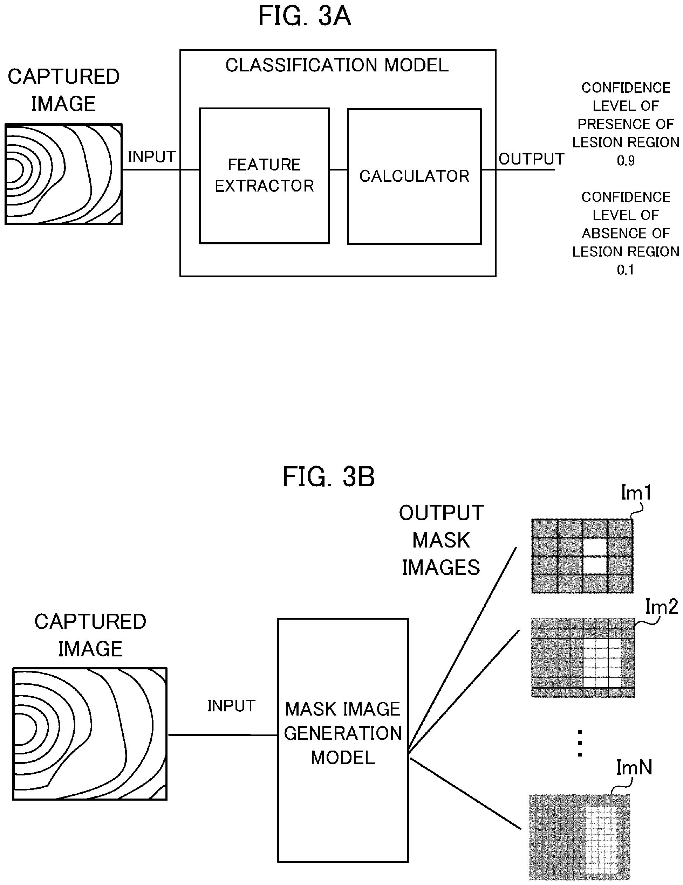

FIG. 3A is a schematic diagram of a classification model configured based on the classification model information D1. In FIG. 3A, a classification model includes a feature extractor and a calculator. The feature extractor extracts features with a predetermined number of dimensions when a captured image Ia is inputted thereto, and supplies the feature vector indicative of the extracted features to the calculator. The calculator outputs the confidence level of the presence of the lesion region and the confidence level of the absence of the lesion region in response to the input of the feature vector from the feature extractor to the calculator.

The classification model may be, for example, a model based on logistic regression, k-neighborhood method, boosting, decision tree, neural network, support vector machine, or the like. . Examples of the architecture of the neural network include a AlexNet, VGG, ResNet, SqueezeNet, DenseNet, Inception, GoogLeNet, ShuffleNet, MobileNet, ResNeXt, Wide ReNet, and NASNet. When the classification model is based on a neural network, the classification model information D1 includes various parameters such as a layer structure, a neuron structure of each layer, the number of filters and the size of filters in each layer, and the weight for each element of each filter.

The mask image generation model information D2 is information regarding a mask image generation model configured to generate a mask image that is an image indicating a biopsy part in a captured image Ia, and includes parameters required for configuring the mask image generation model. The mask image generation model is a machine learning model or a statistical model that is trained to output a plurality of mask images indicating candidate regions for a biopsy part in the inputted captured image Ia with different levels of granularity (i.e., resolutions) when a captured image Ia is inputted thereto. When the mask image generation model is configured by the neural network, the mask image generation model information D2 includes various parameters such as a layer structure, a neuron structure of each layer, the number of filters and the size of filters in each layer, and the weight for each element of each filter.

In the present example embodiment, as an example, the mask image generation model has a network architecture based on Feature Pyramid Networks. In this case, the mask image generation model generates multi-scale feature maps (i.e., tensors acquired by convolution) by performing a convolution on the captured image Ia that is an input image, and infers (i.e., generates mask images with different levels of granularity in the present example embodiment) the biopsy part for each of the feature maps. The number of mask images to be outputted by the mask image generation model and the level of granularity (resolution) of each mask image are preset in the learning stage. Then, such a mask image generation model is learned in advance based on training data (training dataset), and the learned parameters are stored in the mask image generation model information D2. The training data is sets of a plurality of mask images that are used as correct answer data and a captured image that is used as an input image. In learning, for example, the parameters of the mask image generation model are determined by the gradient descent method, the error back propagation method, or the like so that the error (loss) between the output by the mask image generation model when the input image is inputted thereto and the correct answer data is minimized.

It is noted that the mask image generation model is not limited to Feature Pyramid Networks and may be any other learning model configured to output mask images having different levels of granularity when an image is inputted thereto. Examples of architectures of such a neural network include the Featurized Image Pyramid Network, which is configured to perform an inference on plural images which are generated by resizing an inputted captured image Ia to a plurality of resolutions.

FIG. 3B is a schematic diagram of the mask image generation model configured on the basis of the mask image generation model information D2. The mask image generation model outputs a plurality of mask images "Im1" to "ImN" (N is an integer of 2 or more) with different levels of granularity when a captured image Ia is inputted thereto. The mask images Im1 to ImN each is an image showing the reliability of the biopsy part for each grid. Here, each grid is a virtual region acquired by dividing the captured image Ia into grid shapes, and is a rectangular area corresponding to one or more pixels of the captured image Ia. Each of the mask images Im1 to ImN may be an image showing a heat map (reliability map) of the reliability described above on the captured image Ia, or may be a binary image indicating whether or not each grid corresponds to a biopsy part. In the present example embodiment, the term "score" indicates the value of each grid of the mask image. The score shall increase with an increase in the confidence level of the presence of the biopsy part. In the figure, the higher the score is, the closer to white the displayed color become.

Then, the mask images Im1 to ImN have different levels of granularity (resolutions) indicating the reliability of the presence of the biopsy part, and have the numbers of grids depending on the levels of granularity. For example, the mask image Im1 is an image with the 4-by-4 grids in the vertical and horizontal directions, and the mask image Im2 is an image with the 8-by-8 grids in the vertical and horizontal directions.

### (4) Functional Blocks

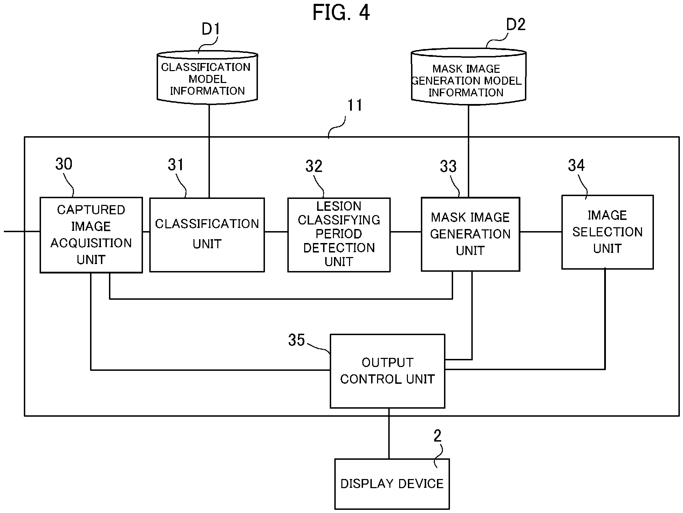

FIG. 4 is a functional block diagram of an image processing device 1 that performs output control related to a biopsy part. The processor 11 of the image processing device 1 functionally includes a captured image acquisition unit 30, a classification unit 31, a lesion classifying period detection unit 32, a mask image generation unit 33, an image selection unit 34, and an output control unit 35. In FIG. 4, blocks to exchange data with each other are connected by a solid line, but the combination of blocks to exchange data is not limited to FIG. 4. The same applies to the drawings of other functional blocks described below.

The captured image acquisition unit 30 acquires the captured image Ia captured by the endoscope 3 via the interface 13 at predetermined intervals. Then, the captured image acquisition unit 30 supplies the acquired captured image Ia to the classification unit 31 and the output control unit 35, respectively.

The classification unit 31 classifies each captured image Ia acquired by the captured image acquisition unit 30 according to whether or not it includes a lesion region, and supplies the classification result to the lesion classifying period detection unit 32. In this instance, the classification unit 31 configures the classification model by referring to the classification model information D1, and acquires the classification result by inputting the captured image Ia acquired by the captured image acquisition unit 30 to the configured classification model.

The lesion classifying period detection unit 32 detects, based on the classification result for each captured image Ia supplied from the classification unit 31, a period of time (also referred to as "lesion classifying period") in which captured images Ia classified as the presence of a lesion region are acquired consecutively. Then, the lesion classifying period detection unit 32 supplies the period detection result regarding the lesion classifying period to the mask image generation unit 33.

The mask image generation unit 33 extracts captured images Ia (also referred to as "candidate images Iac") belonging to the lesion classifying period from the captured images Ia acquired by the captured image acquisition unit 30, based on the period detection result supplied from the lesion classifying period detection unit 32. Then, the mask image generation unit 33 generates N mask images for each candidate image Iac. In this instance, the mask image generation unit 33 configures the mask image generation model by referring to the mask image generation model information D2, and acquires N mask images corresponding to the inputted candidate image Iac by inputting each candidate image Iac to the configured mask image generation model. The mask image generation unit 33 supplies the mask images generated for each candidate image Iac to the image selection unit 34. The candidate images Iac which belong to the lesion classifying period is an example of "time series images".

The image selection unit 34 selects an image for output use (also referred to as "output image Io") from the candidate images Iac based on the mask images for each candidate image Iac supplied from the mask image generation unit 33 and supplies the selected output image Io to the output control unit 35. Here, the "output image Io" is an image to be outputted as an image representing the biopsy part, examples of the output will be described later. In this instance, the image selection unit 34 makes the similarity determination among the mask images, for each candidate image Iac, and determines the output image Io based on the similarity determination. Specifically, the image selection unit 34 first specifies image groups (clusters) of similar mask images for each candidate image Iac, and counts the number (also referred to as "number Nm of the similar mask images") of mask images belonging to the largest cluster, for each candidate image Iac. Then, the image selection unit 34 considers the candidate image Iac having the largest number Nm of the similar mask images as an image in which the biopsy part is stably detected regardless of the levels of granularity of the mask images and selects it as the output image Io.

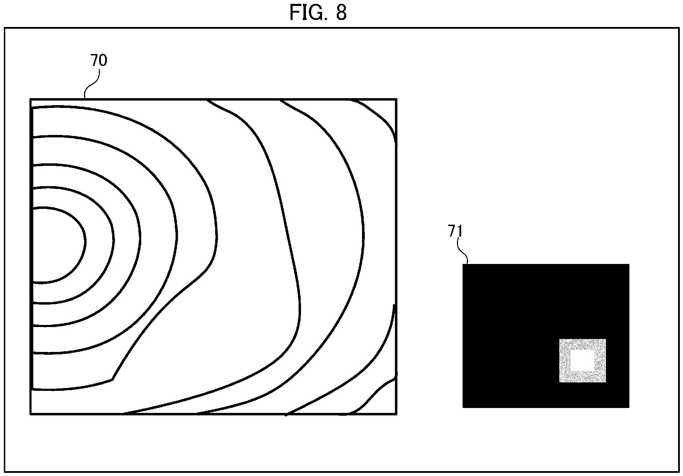

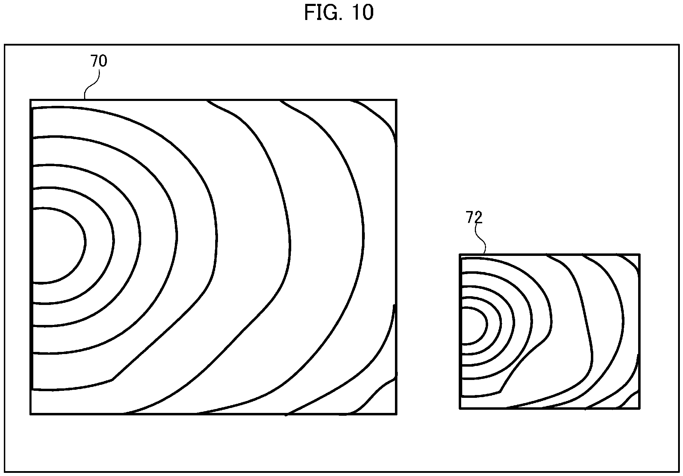

The output control unit 35 generates the display information Ib based on the most recent captured image Ia supplied from the captured image acquisition unit 30 and the output image Io supplied from the image selection unit 34, and supplies the generated display information Ib to the display device 2, thereby causing the display device 2 to display information on the candidate region for the biopsy part. In this instance, for example, the output control unit 35 generates an image in which the candidate region for the biopsy part in the output image Io is highlighted, and displays the generated image on the display device 2 together with the most recent captured image Ia. In this instance, the output control unit 35 may perform a process of specifying the candidate region for the biopsy part using the mask images of the output image Io generated by the mask image generation unit 33. In another example embodiment, the output control unit 35 may use the output image Io as a captured image indicating the biopsy part and display the output image Io as it is on the display device 2 together with the most recent captured image Ia. The display examples displayed by the output control unit 35 on the display device 2 will be described later with reference to FIGS.8 and 10.

By having such a functional configuration as shown in FIG. 4, the image processing device 1 can select an image precisely representing a biopsy part as an output image Io, even when images captured during the endoscopic examination include images with shine, images noise due to splash, and/or blurred images. Then, by presenting the high-precision information regarding the biopsy part based on the output image Io to the examiner, the image processing device 1 can prompts an efficient and effective biopsy, while reducing the labor of the doctor that is an examiner, the burden of the patient, and the cost required for the biopsy test.

Here, for example, each component of the captured image acquisition unit 30, the classification unit 31, the lesion classifying period detection unit 32, the mask image generation unit 33, the image selection unit 34 and the output control unit 35 can be realized by the processor 11 executing a program. In addition, the necessary program may be recorded in any non-volatile storage medium and installed as necessary to realize the respective components. In addition, at least a part of these components is not limited to being realized by a software program and may be realized by any combination of hardware, firmware, and software. At least some of these components may also be implemented using user-programmable integrated circuitry, such as FPGA (Field-Programmable Gate Array) and microcontrollers. In this case, the integrated circuit may be used to realize a program for configuring each of the above-described components. Further, at least a part of the components may be configured by a ASSP (Application Specific Standard Produce), ASIC (Application Specific Integrated Circuit) and/or a quantum processor (quantum computer control chip). In this way, each component may be implemented by a variety of hardware. The above is true for other example embodiments to be described later. Further, each of these components may be realized by the collaboration of a plurality of computers, for example, using cloud computing technology.

### (5) Detection of Lesion Classifying Period

Next, a specific example of a method of detecting a lesion classifying period by the lesion classifying period detection unit 32 will be described.

The lesion classifying period detection unit 32 determines the lesion classifying period to be a period of time in which the classification model has stably classified the captured images as images including a lesion region, based on the classification result outputted by the classification model. Specifically, the lesion classifying period detection unit 32 determines the lesion classifying period to be a period of time in which the condition for each classification of the time-series captured images Ia has consecutively been satisfied. Thus, the lesion classifying period detection unit 32 can determine the period of time, which includes images with little blur caused by the endoscopic operation by the examiner, as the lesion classifying period.

In this case, in the first example, if there are a predetermined number "M" (M is an integer of 2 or more) of consecutive captured images Ia in which each confidence level regarding the presence of a lesion part is equal to or larger than a predetermined first threshold value and the difference in the confidence levels regarding the presence of the lesion part is smaller than a predetermined second threshold value, the lesion classifying period detection unit 32 determines that the M consecutive captured images Ia are captured images Ia which belong to the lesion classifying period. The above-described first threshold value, the second threshold value and the number M are determined in advance in consideration of the classification accuracy of the classification model, for example, and they are stored in advance in the memory 12 or the like.

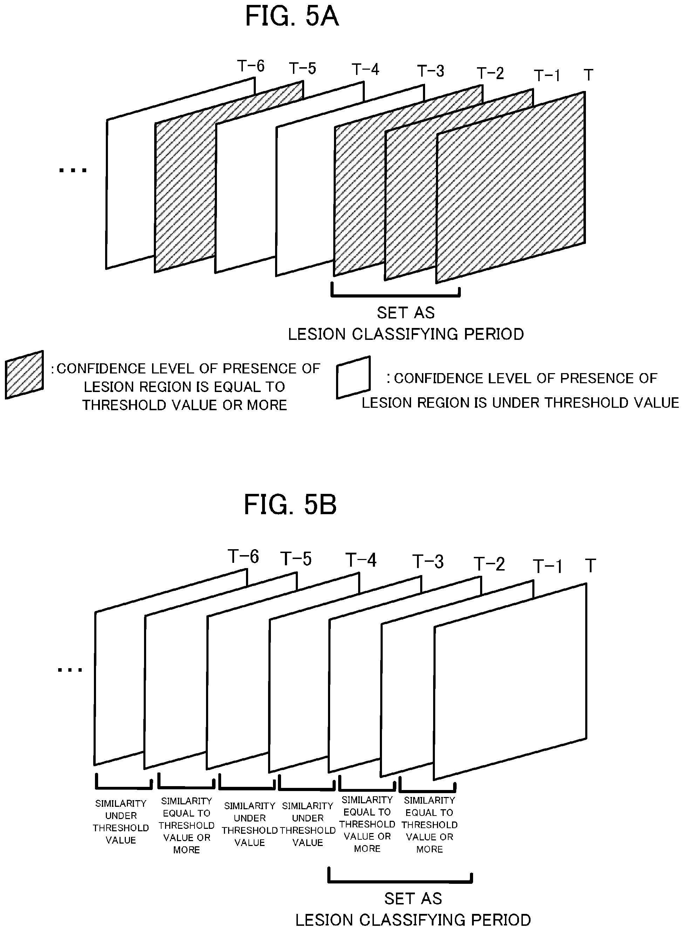

FIG.5A is an outline diagram of the determination method of the lesion classifying period based on the first example. Here, the most recent captured image Ia acquired at the time "T" and the six captured images Ia acquired immediately before the most recent captured image Ia are displayed in association with the acquired times "T" to "T - 6". In addition, any captured mages Ia in which the confidence level of the presence of the lesion region is equal to or larger than the first threshold value are hatched. Here, the number M is assumed to be set to "3". In this instance, for any of the captured image Ia acquired at time T, the captured image Ia acquired at time "T-1", and the captured image Ia acquired at time "T-2", the confidence level of the presence of the lesion region is equal to or larger than the first threshold value. In addition, the difference in the confidence levels of these captured images Ia (that is, the difference between the largest value and the smallest value of the confidence level of these images) is smaller than the second threshold value. Therefore, the lesion classifying period detection unit 32 determines that each captured image Ia acquired at the time T-2, the time T-1, and the time T belong to the lesion classifying period.

In the second example, the lesion classifying period detection unit 32 may determine the lesion classifying period St based on the feature vectors outputted by the feature extractor of the classification model. For example, the lesion classifying period detection unit 32 calculates, based on the inner product of the feature vectors of two consecutive captured image Ia, the degree of similarity (e.g., cosine similarity) between the consecutive captured images Ia. Then, the lesion classifying period detection unit 32 calculates the degrees of similarity for M consecutive captured images Ia. Then upon determining that the degrees of similarity each is equal to or larger than a predetermined threshold value (third threshold value), the lesion classifying period detection unit 32 determines that the M consecutive captured images Ia belong to the lesion classifying period.

FIG. 5B is a schematic diagram of a method for determining a lesion classifying period based on a second example. Here, the most recent captured image Ia acquired at time T and the six captured images Ia acquired at the time T - 1 to time T - 6 immediately before the most recent captured image Ia are displayed in association with the acquired times. In addition, the number M is assumed to be set to "3". In FIG. 5B, the degree of similarity based on the inner product of the feature vectors is calculated for any pairs of two consecutive captured images Ia. Then, the image selection unit 34 compares the calculated degree of similarity with the third threshold value, and determine whether or not there are M consecutive captured images Ia in which the difference in the confidence levels regarding the presence of the lesion part is smaller than the third threshold value. Since the degree of similarity between the captured images Ia acquired at the time T - 2 and time T - 1, and the degree of similarity between the captured image Ia acquired at the time T - 1 and time T became both equal to or larger than the third threshold value, the lesion classifying period detection unit 32 determines that M consecutive captured images Ia acquired at the time T - 2 to time T belong to the lesion classifying period.

The lesion classifying period detection unit 32 may determine the lesion classifying period based on any method other than the first example and the second example described above.

### (6) Selection of Output Image

Next, the processes executed by the mask image generation unit 33 and the image selection unit 34 will be specifically described.

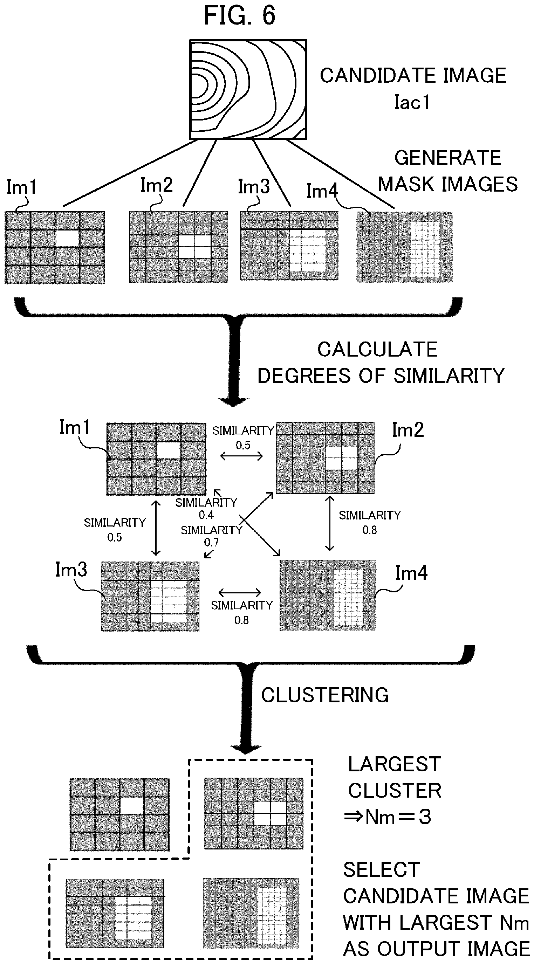

FIG. 6 is a schematic diagram of processes that are executed by the mask image generation unit 33 and the image selection unit 34. Here, as a typical example, it illustrates an outline of the process on the candidate image Iac1 which is one of the candidate images Iac belonging to the lesion classifying period.

First, from the candidate image Iac1, the mask image generation unit 33 generates four mask images Im1 to Im4 (that is, N = 4) having different levels of granularity. Here, the mask image Im1 is a 4-by-4 mask image, the mask image Im2 is a 6-by-6 mask image, the mask image Im3 is an 8-by-8 mask image, and the mask image Im4 is a 15-by-15 mask image.

In this instance, the image selection unit 34 calculates the degree of similarity between the mask images generated for each candidate image Iac. Therefore, the image selection unit 34 calculates the degree of similarity for each of all combinations (six pairs in total) of the pair selected from the mask images Im1 to Im4 for the candidate image Iac1. In the example shown in FIG. 6, the degrees of similarity range from 0 to 1, where 1 represents the highest degree of similarity. The calculation of the degree of similarity will be described later.

Next, the image selection unit 34 applies clustering analysis to the mask images for each candidate image Iac on the basis of the calculated degrees of similarity. In the example shown in FIG. 6, the image selection unit 34 regards any mask images having the degree of similarity equal to or larger than 0.6 with each other as elements of the same cluster, and generates the largest cluster having three elements and the other cluster having one element. The clustering method to be applied may be any clustering method such as the shortest distance method, the group averaging method, the Ward's method, the centroid method, the median method, and the x-means method.

Then, the image selection unit 34 considers the number of elements of the largest clusters as the number Nm of the similar mask images and selects the candidate image Zac having the largest number Nm of similar mask images Nm as the output image Io. Thus, the image selection unit 34 can select a candidate image in which the biopsy part is stably detected regardless of the level of granularity of the mask image as the output image Io. In the example shown in FIG. 6, since the number Nm of the similar mask images for the candidate image Iac1 is 3, the candidate image Iac1 is selected as the output image Nm when the number Nm of the similar mask images for any other candidate image Iac is 2 or less.

If there are plural candidate images Iac having the same largest number Nm of the similar mask images, the image selection unit 34 selects, for example, one candidate image Iac randomly extracted from the candidate images Iac having the same largest number Nm of the similar mask images as the output image Io. The image selection unit 34 may select one candidate image Iac extracted on the basis of any criterion determined in advance from the candidate images Iac having the same largest number of the similar mask images Nm as the output image Io.

Next, a description will be given of a method of calculating the degree of similarity. In the present example embodiment, the image selection unit 34 may calculate the degree of similarity between mask images to be the degree of coincidence of the positions (also referred to as the "score maximum positions") at which the score, which is the value of each grid of the mask image, is maximum in the respective mask images, or may calculate the degree of similarity between mask images to be the degree of similarity across the entire image region between mask images.

First, a description will be given of a case where the degree of coincidence of the score maximum positions is defined as the degree of similarity between mask images.

The image selection unit 34 resizes the N mask images for each candidate image Iac so that the number of grids coincide with any other mask images with respect to the horizontal direction and the vertical direction, for example, by using an arbitrary image resizing method. In this case, for example, the image selection unit 34 defines a two-dimensional coordinate system with one of the four corners as the origin in which each mask image has a value range in common, and determines the score of each grid of each mask image after the resizing from the score of each grid of each mask image before the resizing by an arbitrary interpolation method. The image selection unit 34 determines the score maximum position in each mask image after the resizing and calculates the degree of coincidence of the score maximum positions for all _{N}C₂ combinations selected from N mask images. Any index representing the degree of region overlap such as IoU (Intersection over Union) may be used as an index representing the degree of coincidence in this case. Then, the image selection unit 34 determines that any pair of mask images between which the degree of coincidence of the score maximum positions is equal to or larger than a predetermined threshold value (also referred to as "first similarity determination threshold value") is a pair of similar mask images. The first similarity determination threshold value is stored in advance in the memory 12 or the like. For each candidate image Iac, the image selection unit 34 counts the number of elements of the largest cluster of similar mask images as the number Nm of similar mask images. In this way, the image selection unit 34 calculates the degree of similarity between mask images such that the number Nm of the similar mask images for a candidate image Iac increases (i.e., it is more likely to be selected as the output image Io) with increase in the degree of coincidence of the score maximum positions of the mask images with different resolutions of the candidate image Iac.

In some embodiments, for any mask image in which the maximum score is equal to or less than a predetermined lower limit threshold value, the image selection unit 34 may determine that the mask image does not have the score maximum position and is not similar to any other mask images (that is, it does not belong to the largest cluster for counting the number of similar mask image Nm). The lower limit threshold value is determined to be a lower limit value of the score that there is a possibility of existence of a biopsy part, for example, and is previously stored in the memory 12 or the like. Thus, the image selection unit 34 can suppress making such a determination that the mask images of the candidate image Iac in which obviously there is no biopsy part are similar to each other.

Next, a description will be given of a case where the degree of similarity in the entire region between mask images is used as the degree of similarity between the mask images. In this case, the image selection unit 34 calculates the degree of similarity based on any image similarity index such as cosine similarity, MSE (Mean Squared Error), and SSIM (Structural Similarity) for all _{N}C₂ combinations of N mask images. Then, the image selection unit 34 determines that a pair of mask images between which the degree of similarity is equal to or larger than a predetermined threshold value (also referred to as "second similarity determination threshold value") is a pair of similar mask images. The second similarity determination threshold value is stored in advance in the memory 12 or the like. For each candidate image Iac, the image selection unit 34 counts the number of elements of the largest cluster of similar mask images as the number Nm of the similar mask images. In this way, the image selection unit 34 calculates the degree of similarity between mask images such that the number Nm of the similar mask images for a candidate image Iac increases (that is, it is likely to be selected as the output image Io) with an increase in the number of similar mask images of the candidate image Iac with different resolutions.

In some embodiments, even when the degree of similarity across entire image region between mask images is defined as the degree of similarity between the mask images, the image selection unit 34 may determine that any mask images whose maximum score is equal to or less than the predetermined lower limit threshold value are not similar to any other mask images. That is, the image selection unit 34 sets the degree of similarity with respect to each mask image whose maximum score is equal to or less than the predetermined lower limit threshold value, regardless of the calculation result of the degree of similarity, to be less than the second similarity determination threshold value. Thus, the image selection unit 34 can suppress making such a determination that mask images of the candidate image Iac in which obviously there is no biopsy part are similar to each other.

The image selection unit 34 may calculate the number Nm of similar mask images by combining the case where the degree of coincidence of the score maximum positions is defined as the degree of similarity between mask images with the case where the degree of similarity across entire image region between the mask images is defined as the degree of similarity between the mask images. For example, for each possible pair of mask images, the image selection unit 34 calculates both the degree of similarity equivalent to the degree of coincidence of the score maximum positions and the degree of similarity across entire image region between mask images. Then, upon determining that the degree of similarity equivalent to the degree of coincidence of the score maximum positions is equal to or larger than the first similarity determination threshold value, or, the degree of similarity entire images between mask images is equal to or larger than the second similarity determination threshold value, the image selection unit 34 determines that the target pair of the mask images is a pair of similar mask images. Then, for each candidate image Iac, the image selection unit 34 calculates the number of elements of the largest cluster of the similar mask images as the number of similar mask images Nm. In this manner, the image selection unit 34 can determine that a pair of mask images that are similar to each other as a whole, even if the score maximum positions of the pair of mask images are different, is a pair of similar mask images.

In some embodiments, the image selection unit 34 may calculate the number Nm of the similar mask images for a candidate image Iac, considering mask images generated from previous captured image(s) Ia taken prior to the candidate image Iac in addition to the mask images of the candidate image Iac.

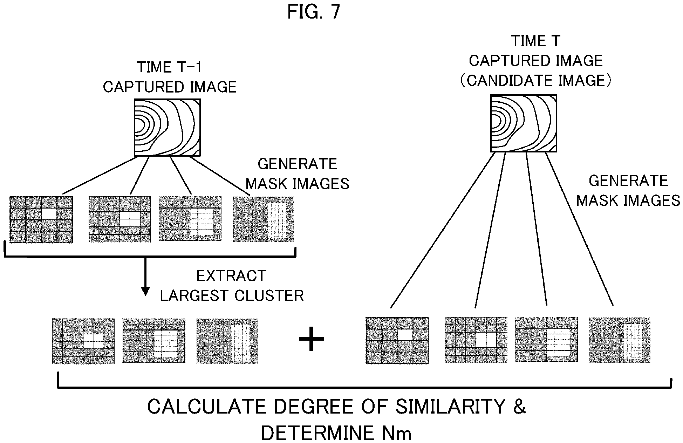

FIG. 7 illustrates an overview of calculating the number Nm of similar mask images using the mask images of the previous captured image Ia. In this example embodiment, in order to calculate the number Nm of the similar mask images for a candidate image Iac which is the captured image Ia at the time T, the mask images generated from the previous captured image Iac, which is captured at the time T - 1 just before the candidate image Iac, is used in addition to the mask images generated from the candidate image Iac. Here, the number of mask images is assumed to be four (N = 4).

In this instance, the image selection unit 34 calculates the degree of similarity between the mask images using the mask images of the candidate image Iac and the mask images of the captured image Ia at the time T - 1, and calculates the number of pairs between which the degree of similarity is equal to or larger than a threshold value as the number Nm of the similar mask images. In this instance, instead of using all of the mask images of the captured image Ia at the time T - 1, the image selection unit 34 uses the mask images (three mask images in this case) that constitutes the largest cluster obtained by clustering all mask images of the captured image Ia at the time T - 1. Therefore, in this case, the image selection unit 34 calculates the degrees of similarity for all combinations ₇C₂ of mask images which include three mask images of the captured image Ia at the time T - 1 and four mask images of the candidate image Iac, and then determines the number Nm of the similarity mask images based on the degrees of similarity. Thus, the image selection unit 34 can reduce an increase in the number of target pairs of calculation of the degree of similarity caused by using all of the mask images of the captured image Ia at the time T-1 and an increase in the computational cost associated therewith. Therefore, while reducing the increase in the computational cost, the image selection unit 34 can calculate the number Nm of the similar mask images such that the number Nm of the similar mask images for a candidate image Iac increases with an increase in the degree of time-series stability of detection of a biopsy part.

Instead of the example shown in FIG. 7, the image selection unit 34 may calculate the number Nm of the similar mask images using one mask image of the largest clusters of the mask images of the captured image Ia at the time T - 1. In this case, the image selection unit 34 calculates the degrees of similarity for respective ₅C₂ combinations selected from mask images which include one mask image of the captured image Ia at the time T - 1 and four mask images of the candidate image Iac, and then determines the number Nm of the similar mask images based on the degrees of similarity. Thus, it is possible to further reduce the number of mask images as a calculation target of the degree of similarity to thereby reduce the calculation load.

In this case, if the number of the mask images belonging to the largest clusters among the mask images of the captured image Ia at the time T - 1 is equal to or less than a predetermined number, the image selection unit 34 may calculate the number Nm of the similar mask images without using any mask images of the captured image Ia at the time T - 1. Thus, the image selection unit 34 can suppress the calculation of the number Nm of the similar mask images using any mask images of the previous captured image Ia in which a biopsy part cannot be stably detected.

Then, the image selection unit 34 selects the output image Io to be a candidate image Iac having the maximum number Nm of the similar mask images. In this case, in some embodiments, when the maximized number Nm of the similar mask images is equal to or less than a predetermined threshold value, the image selection unit 34 determines that there is no suitable candidate image Iac as the output image Io and does not need to select the output image Io. The above-described threshold value is determined to be, for example, the lower limit of the number Nm of the similar mask images assumed when there is a biopsy part, and is stored in the memory 12 or the like in advance. Thus, the image selection unit 34 can suppress erroneously selecting the output image Io from any candidate images Iac belonging to the lesion classifying period in which a biopsy part does not actually exist.

### (7) Display Based on Output Image

Next, a description will be given of the output control of the display device 2 executed by the output control unit 35.

FIG. 8 shows a first display example of a display screen image displayed by the display device 2 in the endoscopic examination. The output control unit 35 of the image processing device 1 generates the display information Ib on the basis of the captured image Ia acquired by the captured image acquisition unit 30 and the output image Io supplied from the image selection unit 34, then transmit the display information Ib to the display device 2 to thereby cause the display device 2 to display the display screen image according to the first display example.

In the first display example, the output control unit 35 of the image processing device 1 displays the most recent captured image 70 and the biopsy part map 71 on the display screen image. The output control unit 35 displays a moving image based on the most recent captured image Ia acquired by the captured image acquisition unit 30 as the most recent captured image 70. The output control unit 35 displays a biopsy part map 71 which is a mask image representing the biopsy part in the output image Io acquired from the image selection unit 34. The method of generating the biopsy part map 71 will be described later.

In some embodiments, the output control unit 35 may superimpose the display corresponding to the biopsy part map 71 on the most recent captured image 70. In this case, the output control unit 35 may superimpose a heat map based on the biopsy part map 71 on the biopsy part map 71, or may highlight a range in which the value is equal to or more than a predetermined threshold value in the biopsy part map 71 by edging effect.

According to the first display example, the image processing device 1 suitably presents a region to be a candidate for a biopsy part to an examiner, and can support efficient and effective biopsy implementation.

Here, a supplementary description will be given of the display of the biopsy part map 71. The output control unit 35, for example, acquires one or more mask images of the output image Io from the mask image generation unit 33, and displays the biopsy part map 71 based on the mask images. In this instance, the output control unit 35 displays the biopsy part map 71 through integration or selection of mask image(s) of the output image Io.

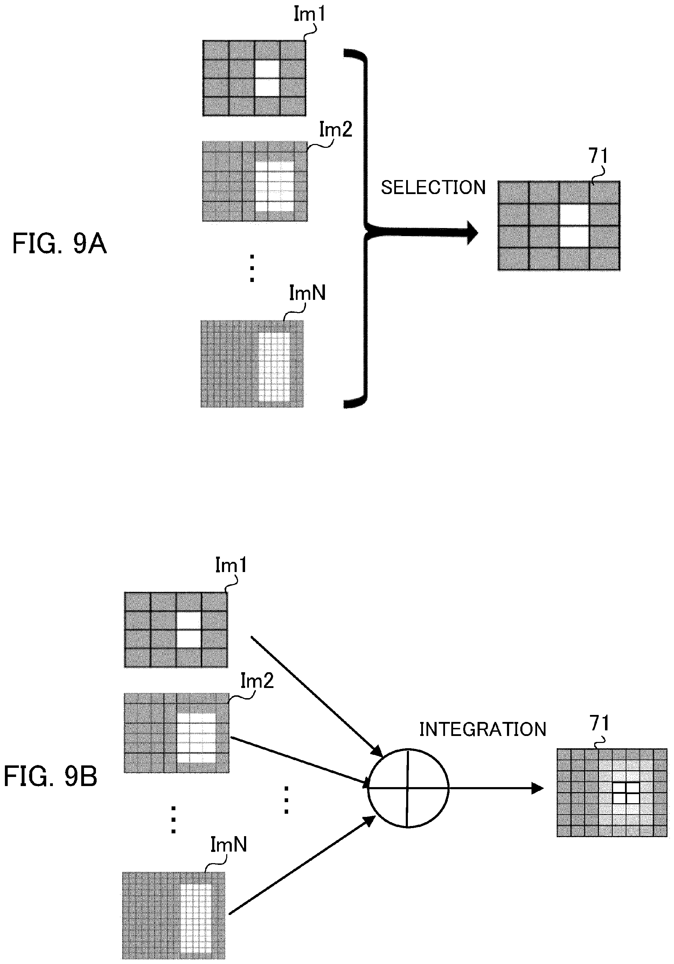

FIG. 9A illustrates an outline of a process of displaying one mask image as the biopsy part map 71 selected from the mask images Im1 to ImN of the output image Io. For example, the output control unit 35 specifies the maximum score for each mask image and displays the mask image having the largest maximum score as the biopsy part map 71. In another example, the output control unit 35 displays one mask image selected from the mask images belonging to the largest cluster as the biopsy part map 71 on the basis of the clustering result supplied from the image selection unit 34. In addition, the output control unit 35 may display an image selected by any methods from the mask images Im1 to ImN of the output image Io as the biopsy part map 71.

FIG. 9B illustrates an outline of a process of displaying an image obtained by integrating the mask images Im1 to ImN of the output image Io as the biopsy part map 71. In this instance, the output control unit 35 resizes the mask images Im1 to ImN so as to have a common resolution (same level of granularity), and displays an image obtained by averaging (or summing up) the resized mask images Im1 to ImN with respect to each corresponding grid as the biopsy part map 71. In the example shown in FIG. 9B, the output control unit 35 converts the mask images Im1 to ImN into mask images having a level of granularity (the number of grids) of 8-by-8, and then generates an image having a level of granularity of 8-by-8 obtained by integrating the mask images after the conversion. The output control unit 35 may select target mask images of integration from the mask images Im1 to ImN. For example, the output control unit 35 may select only mask images belonging to the largest cluster as the target mask images of integration, based on the clustering result supplied from the image selection unit 34.

The method of generating the biopsy part map 71 is not limited to the method of integrating or selecting mask image(s) of the output image Io. In another example, the output control unit 35 may input the output image Io to a segmentation model configured to extract (segment) a region corresponding to the biopsy part such as a lesion region from an image inputted thereto, and generate the biopsy part map 71 based on the result outputted by the segmentation model in response to the input. In this case, the information that the segmentation model outputs may be, for example, a binary image indicative of the presence or absence of a biopsy part for each grid, or may be a reliability map showing the reliability of the presence of a biopsy part on a scale of three or more levels. Examples of the typical model of the neural network used in the segmentation model include Fully Convolutional Network, SegNet, U-Net, V-Net, Feature Pyramid Network, Mask R-CNN, and DeepLab. The parameters of the segmentation model are stored in advance in the memory 12 or the like.

FIG. 10 shows a second display example of a display screen image displayed by the display device 2 in the endoscopic examination. The output control unit 35 of the image processing device 1 generates the display information Ib based on the output image Io supplied from the image selection unit 34 and the captured image Ia acquired by the captured image acquisition unit 30 and then transmit the display information Ib to the display device 2 to thereby display the display screen image according to the second display example on the display device 2.

In the second display example, the output control unit 35 of the image processing device 1 displays the most recent captured image 70 and the captured image 72 on the display screen image. The output control unit 35 displays a moving image based on the most recent captured image Ia acquired by the captured image acquisition unit 30 as the most recent captured image 70. The output control unit 35 displays the most recent output image Io acquired from the image selection unit 34 as the captured image 72. The output control unit 35 updates the captured image 72 so as to display the most recent output image Io each time a new output image Io is acquired from the image selection unit 34. Thus, in the second display example, the output control unit 35 displays the captured image 72, which is the output image Io selected as the captured image Ia representing the candidate for the biopsy part, together with the most recent captured image 70. Thereby, it enables the examiner to confirm the presence of the candidate for a biopsy part.

### (8) Processing Flow

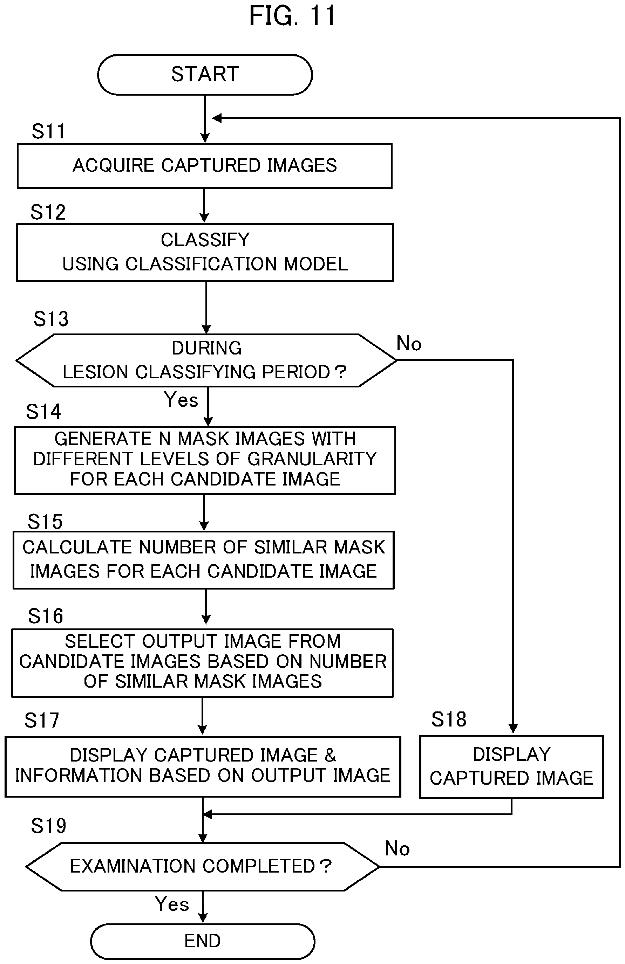

FIG. 11 is an example of a flowchart illustrating an outline of a process that is executed by the image processing device 1 during the endoscopic examination in the first example embodiment.

First, the captured image acquisition unit 30 of the image processing device 1 acquires captured images Ia (step S11). In this instance, the captured image acquisition unit 30 of the image processing device 1 receives captured images Ia from the endoscope 3 via the interface 13.

Next, the classification unit 31 of the image processing device 1 classifies the captured images Ia acquired at step S11 by the classification model configured on the basis of the classification model information D1 (step S12). The lesion classifying period detection unit 32 of the image processing device 1 determines, based on the classification result generated by the classification unit 31, whether or not it is in the lesion classifying period (step S13). Then, if it is in the lesion classifying period (step S13; Yes), the process proceeds to the process at step S14. On the other hand, if it is not the lesion classifying period (step S13; No), the output control unit 35 displays the most recent captured image Ia acquired at step S11 on the display device 2 (step S18).

At step S14, the mask image generation unit 33 of the image processing device 1 generates N mask images with different levels of granularity for each of candidate images Ia which are captured images Iac acquired during the lesion classifying period (step S14). Then, the image selection unit 34 of the image processing device 1 calculates the number Nm of the similar mask images for each of the candidate images Iac by making the similarity determination between the mask images generated at step S14 (step S15).

Next, the image selection unit 34 of the image processing device 1 selects the output image Io from the candidate images Iac based on the number Nm of the similar mask images (step S16). Then, the output control unit 35 displays the captured image Ia acquired at step S11 and the information based on the output image Io on the display device 2 (step S17). In this instance, the output control unit 35 causes the display device 2 to display an image based on the output image Io or the output image Io as it is as information regarding the biopsy part. Thus, the output control unit 35 can present the existence and position of the identified biopsy part to the user.

Then, the image processing device 1 determines whether or not the endoscopic examination has been completed after the process at step S17 or the process at step S18 (step S19). For example, the image processing device 1 determines that the endoscopic examination has been completed if a predetermined input or the like to the input unit 14 or the operation unit 36 is detected. Upon determining that the endoscopic examination has been completed (step S19; Yes), the image processing device 1 ends the process of the flowchart. On the other hand, upon determining that the endoscopic examination has not been completed (step S19; No), the image processing device 1 gets back to the process at step S11. Then, the image processing device 1 performs the processes at step S11 to step S19 using a captured image Ia newly generated by the endoscope 3.

### (9) Modifications

Next, modifications applicable to the above-described example embodiment will be described. The following modifications may be applied to the example embodiments described above in any combination.

### (First Modification)

The image processing device 1 may process, after the examination, a video configured by captured images Ia that were generated during endoscopic examination.

For example, when the video to be processed is designated based on a user input by the input unit 14 at any timing after the examination, the image processing device 1 sequentially applies the procedure of the flowchart shown in FIG. 11 to the time series captured images Ia constituting the video. Then, the image processing device 1 ends the process of the flowchart when it is determined at step S19 that the video has ended. In contrast, if the video has not ended, the image processing device 1 gets back to the process at step S11 to continue the process of the flowchart for the subsequent captured image Ia.

### (Second Modification)

The classification model information D1 and the mask image generation model information D2 may be stored in a storage device separate from the image processing device 1.

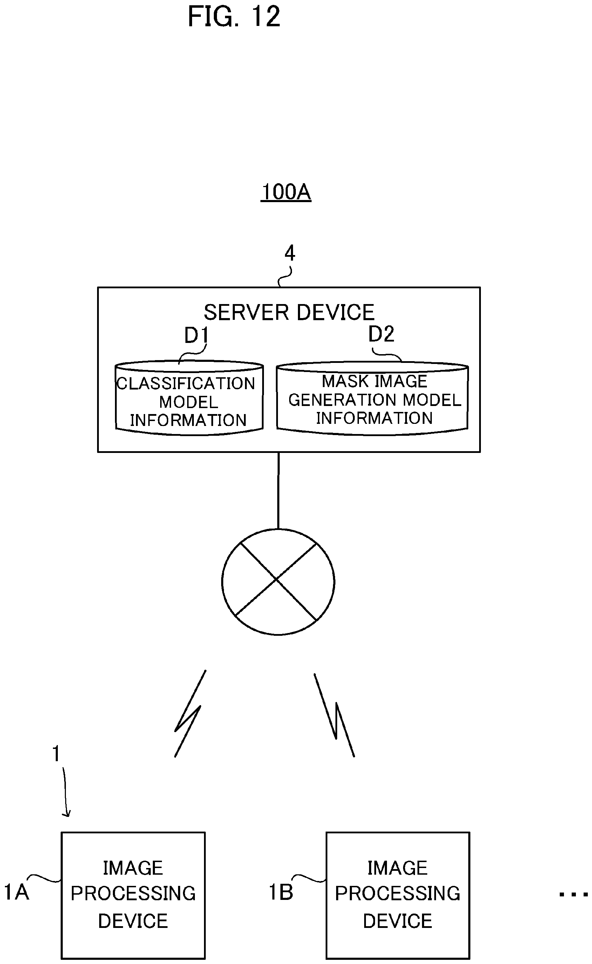

FIG. 12 is a schematic configuration diagram of an endoscopic examination system 100A according to the second modification. For simplicity, the display device 2 and the endoscope 3 and the like are not shown. The endoscopic examination 100A includes a server device 4 that stores the classification model information D1 and the mask image generation model information D2. Further, the endoscopic examination system 100A includes a plurality of image processing devices 1 (1A, 1B, ...) capable of data communication with the server device 4 via a network.

In this instance, the image processing device 1 refers to the classification model information D1 and the mask image generation model information D2 through the network. In this case, the interface 13 of each image processing device 1 includes a communication interface such as a network adapter for performing data communication. In this configuration, the image processing devices 1 can suitably perform the display processing relating to the biopsy part by referring to the classification model information D1 and the mask image generation model information D2 as in the above-described example embodiment.

### (Third Modification)

The image processing device 1 may use a classification model configured to perform classification into three or more classes, instead of the classification model (i.e., binary classification model) configured to classify the presence or absence of a lesion part in the captured image Ia. For example, the classification model to be used by the image processing device 1 may be a model configured to perform classification into "X + 1" classes "first lesion type" to "Xₜₕ lesion type" ("X" is an integer of two or more) and "non-lesion". In this instance, the memory 12 stores, as the classification model information D1, classification model information that is information regarding the classification model configured to perform classification into three or more classes. The image processing device 1 generates the classification result for the captured image Ia by using the classification model with reference to the classification model information. Thus, the classification model to be used by the image processing device may be a model configured to determine "presence or absence of a lesion", and is not limited to the binary classification model.

### (Fourth Modification)

The detection target to be detected by the mask generation model is not limited to a biopsy part, and it may be any attention part (point) that the examiner needs to notice. Examples of such an attention part include a lesion part, an inflammation part, a point with an operating mark or other cuts, a point with a fold or a protrusion, a point on the wall surface of the lumen where the tip unit 38 of the endoscope 3 tends to get contact (caught).

The mask image generation model is trained to output, when the captured image Ia is inputted thereto, a mask image in which a predetermined attention part is indicated by different levels of granularity. Then, the mask image generation unit 33 of the image processing device 1 generates N mask images regarding the attention part from the captured image Ia using such a mask image generation model, the image selection unit 34 selects the output image Io based on the mask images, and the output control unit 35 displays information relating to the attention part based on the output image Io. Thus, the image processing device 1 can suitably present the attention part to the examiner. The present modification also achieves such an effect equivalent to the effect of the biopsy part extraction described in the first example embodiment mentioned before this modification.

### (Fifth Modification)

The image processing device 1 may select the output image Io without processing executed by the classification unit 31 and the lesion classifying period detection unit 32.

In this instance, for example, the image processing device 1 uses the most recent M captured images Ia acquired by the captured image acquisition unit 30 as the candidate images Iac and selects the output image Io from the candidate images Iac based on the mask images of the respective candidate images Iac. Then, the image processing device 1 selects the most recent M captured images Ia and selects the output image Io at predetermined time intervals. In this case, for example, when the maximum number Nm of the similar mask images is equal to or less than a predetermined threshold value, the image processing device 1 determines that there is no suitable candidate image Iac as the output image Io and does not select the output image Io. Thus, the image selection unit 34 can suppress selecting the output image Io erroneously in the period of time in which the biopsy part does not actually exist. In this modification, as described in the section "(6) Selection of Output Image", the image processing device 1 may determine that mask images whose maximum score is equal to or less than the predetermined lower limit threshold value is not similar to any other mask images. Thus, the image selection unit 34 can suppress making a determination that mask images of the candidate image Iac in which obviously there is no biopsy part are similar to each other. In this modification, the most recent M captured image Ia captured by the captured image acquisition unit 30 is an example of "time series images".

### <Second Example Embodiment>

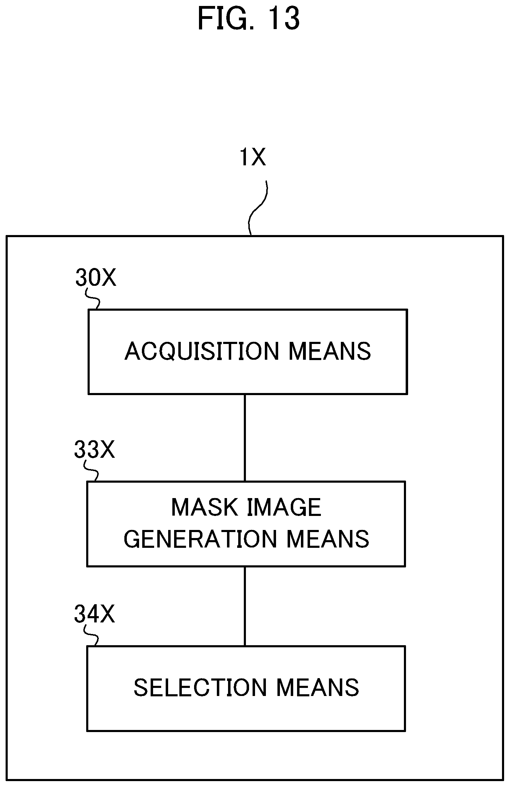

FIG. 13 is a block diagram of an image processing device 1X according to the second example embodiment. The image processing device 1X includes an acquisition means 30X, a mask image generation means 33X, and a selection means 34X. The image processing device 1X may be configured by a plurality of devices.

The acquisition means 30X is configured to acquire time series images obtained by photographing an examination target by a photographing unit provided in an endoscope. Examples of the acquisition means 30X include the captured image acquisition unit 30 in the first example embodiment (including modifications, hereinafter the same). The acquisition means 30X may immediately acquire the captured images generated by the photographing unit, or may acquire, at a predetermined timing, the captured images stored in the storage device generated by the photographing unit in advance.

The mask image generation means 33X is configured to generate a plurality of mask images, which indicate candidate regions for an attention part with different levels of granularity, for each of the time series images. The "mask image" is not limited to an image which indicates whether or not there is an attention part per pixel by two values, but may be an image which indicates the reliability of presence of an attention part on a scale of three or more levels. Examples of the mask image generation means 33X include the mask image generation unit 33 in the first example embodiment.

The selection means 34X is configured to select an output image for output use from the time series images, based on the plurality of mask images. Here, the "output" may be a display of the output image, or may be an output of the output image into a model configured to perform image segmentation regarding an attention part. Examples of the "display of the output image" include not only displaying the output image as it is but also displaying the processed output image. Examples of the selection means 34X include the image selection unit 34 in the first example embodiment.

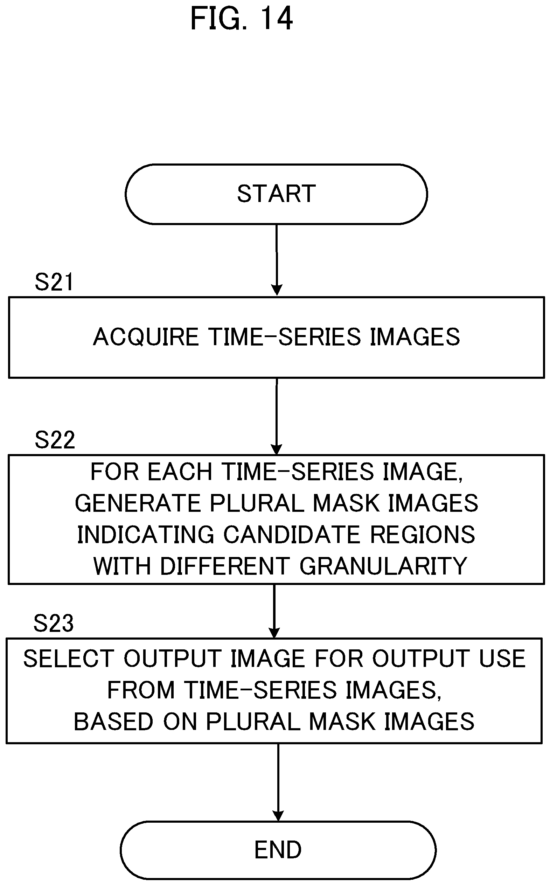

FIG. 14 is an example of a flowchart showing a processing procedure in the second example embodiment. The acquisition means 30X acquires time series images obtained by photographing an examination target by a photographing unit provided in an endoscope (step S21). The mask image generation means 33X generates a plurality of mask images, which indicate candidate regions for an attention part with different levels of granularity, for each of the time series images (step S22). Then, the selection means 34X selects an output image for output use from the time series images, based on the plurality of mask images (step S23).

According to the second example embodiment, the image processing device 1X can appropriately select an output image to be an image that accurately represents an attention part from captured images obtained by photographing the examination target.

The whole or a part of the example embodiments described above (including modifications, the same applies hereinafter) can be described as, but not limited to, the following Supplementary Notes.

### [Supplementary Note 1]

An image processing device comprising:
an acquisition means configured to acquire time series images obtained by photographing an examination target by a photographing unit provided in an endoscope;
a mask image generation means configured to generate a plurality of mask images, which indicate candidate regions for an attention part with different levels of granularity, for each of the time series images; and
a selection means configured to select an output image for output use from the time series images, based on the plurality of mask images.

### [Supplementary Note 2]

The image processing device according to Supplementary Note 1,
wherein the selection means is configured to
make a similarity determination among the plurality of mask images, for each of the time series images and
select the output image based on a result of the similarity determination.

### [Supplementary Note 3]

The image processing device according to Supplementary Note 2,
wherein the selection means is configured to
count a number of similar mask images, which is a number of images of a largest group of the similar mask images among the plurality of mask images, for each of the time series images, and
select the output image based on the number of the similar mask images.

### [Supplementary Note 4]

The image processing device according to Supplementary Note 2 or 3,
wherein the selection means is configured to
determine a score maximum position, at which a score of reliability of presence of the attention part per grid is maximum, for each of the plurality of mask images, and
make the similarity determination based on the score maximum position.

### [Supplementary Note 5]

The image processing device according to any one of Supplementary Notes 2 to 4,
wherein the selection means is configured to
calculate degrees of similarity across entire image region among the plurality of mask images and
make the similarity determination based on the degrees of similarity.

### [Supplementary Note 6]

The image processing device according to any one of Supplementary Notes 2 to 5,
wherein the selection means is configured to exclude a mask image, in which a maximum value of a score of reliability of presence of the attention part per grid is equal to or smaller than a predetermined value, from a target of the similarity determination.

### [Supplementary Note 7]

The image processing device according to any one of Supplementary Notes 1 to 6, further comprising an output control means configured to output information regarding the output image.

### [Supplementary Note 8]

The image processing device according to Supplementary Note 7,
wherein the output control means is configured to display the output image or an image based on the output image on a display device.

### [Supplementary Note 9]

The image processing device according to Supplementary Note 7 or 8,
wherein the output control means is configured to input the output image to a segmentation model and display, on a display device, information outputted by the segmentation model accordingly,
wherein the segmentation model is a model configured to output, when an image is inputted thereto, information regarding a candidate region for an attention part in the inputted image.

### [Supplementary Note 10]

The image processing device according to Supplementary Note 7 or 8,
wherein the output control means is configured to display, on a display device, an image indicating a candidate area for the attention part based on the mask images associated with the output image.

### [Supplementary Note 11]

The image processing device according to any one of Supplementary Notes 1 to 10,
wherein the attention part is a part for a biopsy.

### [Supplementary Note 12]

An image processing method executed by a computer, the image processing method comprising:
acquiring time series images obtained by photographing an examination target by a photographing unit provided in an endoscope;
generating a plurality of mask images, which indicate candidate regions for an attention part with different levels of granularity, for each of the time series images; and
selecting an output image for output use from the time series images, based on the plurality of mask images.

### [Supplementary Note 13]

A storage medium storing a program executed by a computer, the program causing the computer to:
acquire time series images obtained by photographing an examination target by a photographing unit provided in an endoscope;
generate a plurality of mask images, which indicate candidate regions for an attention part with different levels of granularity, for each of the time series images; and
select an output image for output use from the time series images, based on the plurality of mask images.

While the invention has been particularly shown and described with reference to example embodiments thereof, the invention is not limited to these example embodiments. It will be understood by those of ordinary skilled in the art that various changes in form and details may be made therein without departing from the spirit and scope of the present invention as defined by the claims. In other words, it is needless to say that the present invention includes various modifications that could be made by a person skilled in the art according to the entire disclosure including the scope of the claims, and the technical philosophy. All Patent and Non-Patent Literatures mentioned in this specification are incorporated by reference in its entirety.

### DESCRIPTION OF REFERENCE NUMERALS

- 1, 1A, 1B, 1X: Image processing device
- 2: Display device
- 3: Endoscope
- 11: Processor
- 12: Memory
- 13: Interface
- 14: Input unit
- 15: Light source unit
- 16: Audio output unit
- 100, 100A: Endoscopic examination system

## Claims

1. An image processing device comprising:
an acquisition means configured to acquire time series images obtained by photographing an examination target by a photographing unit provided in an endoscope;
a mask image generation means configured to generate a plurality of mask images, which indicate candidate regions for an attention part with different levels of granularity, for each of the time series images; and
a selection means configured to select an output image for output use from the time series images, based on the plurality of mask images.

2. The image processing device according to claim 1,
wherein the selection means is configured to
make a similarity determination among the plurality of mask images, for each of the time series images and
select the output image based on a result of the similarity determination.

3. The image processing device according to claim 2,
wherein the selection means is configured to
count a number of similar mask images, which is a number of images of a largest group of the similar mask images among the plurality of mask images, for each of the time series images, and
select the output image based on the number of the similar mask images.

4. The image processing device according to claim 2 or 3,
wherein the selection means is configured to
determine a score maximum position, at which a score of reliability of presence of the attention part per grid is maximum, for each of the plurality of mask images, and
make the similarity determination based on the score maximum position.

5. The image processing device according to any one of claims 2 to 4,
wherein the selection means is configured to
calculate degrees of similarity across entire image region among the plurality of mask images and
make the similarity determination based on the degrees of similarity.

6. The image processing device according to any one of claims 2 to 5,
wherein the selection means is configured to exclude a mask image, in which a maximum value of a score of reliability of presence of the attention part per grid is equal to or smaller than a predetermined value, from a target of the similarity determination.

7. The image processing device according to any one of claims 1 to 6, further comprising an output control means configured to output information regarding the output image.

8. The image processing device according to claim 7,
wherein the output control means is configured to display the output image or an image based on the output image on a display device.

9. The image processing device according to claim 7 or 8,
wherein the output control means is configured to input the output image to a segmentation model and display, on a display device, information outputted by the segmentation model accordingly,
wherein the segmentation model is a model configured to output, when an image is inputted thereto, information regarding a candidate region for an attention part in the inputted image.

10. The image processing device according to claim 7 or 8,
wherein the output control means is configured to display, on a display device, an image indicating a candidate area for the attention part based on the mask images associated with the output image.

11. The image processing device according to any one of claims 1 to 10,
wherein the attention part is a part for a biopsy.

12. An image processing method executed by a computer, the image processing method comprising:
acquiring time series images obtained by photographing an examination target by a photographing unit provided in an endoscope;
generating a plurality of mask images, which indicate candidate regions for an attention part with different levels of granularity, for each of the time series images; and
selecting an output image for output use from the time series images, based on the plurality of mask images.

13. A storage medium storing a program executed by a computer, the program causing the computer to:
acquire time series images obtained by photographing an examination target by a photographing unit provided in an endoscope;
generate a plurality of mask images, which indicate candidate regions for an attention part with different levels of granularity, for each of the time series images; and
select an output image for output use from the time series images, based on the plurality of mask images.
